(19) Europäisches Patentamt
European Patent Office
Office européen des brevets

(11) Veröffentlichungsnummer: **0 266 589 B1**

(12) # EUROPÄISCHE PATENTSCHRIFT

(45) Veröffentlichungstag der Patentschrift: **27.12.90**

(51) Int. Cl.[5]: **C07C 271/24**, C07C 271/28, C08F 20/36, A61K 6/083

(21) Anmeldenummer: **87114974.6**

(22) Anmeldetag: **13.10.87**

(54) **(Meth)-Acrylsäure-Derivate von Triisocyanaten und ihre Verwendung.**

(30) Priorität: **24.10.86 DE 3636189**

(43) Veröffentlichungstag der Anmeldung: **11.05.88 Patentblatt 88/19**

(45) Bekanntmachung des Hinweises auf die Patenterteilung: **27.12.90 Patentblatt 90/52**

(84) Benannte Vertragsstaaten: **AT BE CH DE ES FR GB IT LI NL SE**

(56) Entgegenhaltungen:
**EP-A- 0 173 085**
**DE-B- 2 308 036**
**US-A- 4 400 159**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk(DE)**

(72) Erfinder: **Reiners, Jürgen, Dr., Carl-Rumpff-Strasse 57, D-5090 Leverkusen 1(DE)**
Erfinder: **Podszun, Wolfgang, Dr., Wolfskaul 4, D-5000 Köln 80(DE)**
Erfinder: **Süling, Carlhans, Dr., Carl-Leverkus-Strasse 10, D-5068 Odenthal(DE)**
Erfinder: **Winkel, Jens, Dr., Hahnenweg 6, D-5000 Köln 80(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft neue Acrylsäure- und Methacrylsäurederivate von Triisocyanaten, im folgenden (Meth)-Acrylsäure-Derivate genannt, und ihre Herstellung. Die neuen Verbindungen können als Monomere für die Anwendung im Dentalbereich eingesetzt werden.

Aus der DE-B 2 308 036 und der US-P 4 400 159 sind bereits polymerisierbare Acrylat- bzw. Methacrylatgruppen enthaltende, von Oligoisocyanaten abgeleitete, Urethanderivate enthaltende Zahnfüll- bzw. Beschichtungsmaterialien bekannt geworden. Die mechanischen Eigenschaften dieser Dentalwerkstoffe entsprechen jedoch nicht höchsten Anforderungen.

Es wurden neue (Meth)-Acrylsäure-Derivate von Triisocyanaten der Formel

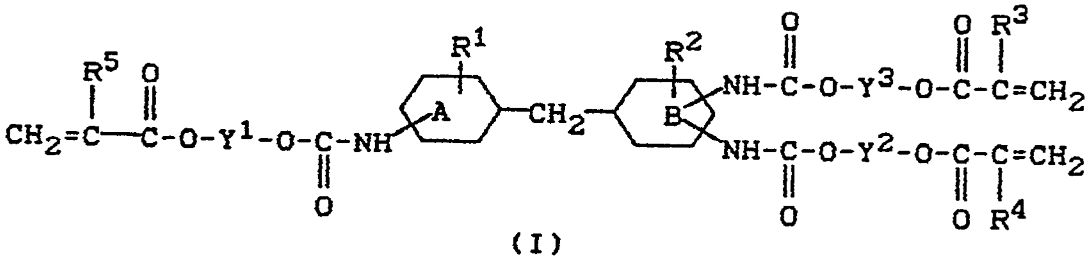

(I)

gefunden, in der $R^1$ und $R^2$ gleich oder verschieden sind und für Wasserstoff oder einen Alkylrest mit 1-6 Kohlenstoffatomen stehen,

$R^3$, $R^4$ und $R^5$ gleich oder verschieden sind und Wasserstoff oder Methyl bedeuten, $Y^1$ bis $Y^3$ gleich oder verschieden sind und zweiwertige geradkettige oder verzweigte aliphatische Kohlenwasserstoffreste mit 2 bis 15 Kohlenstoffatomen, die gegebenenfalls 1 bis 3 Sauerstoffbrücken enthalten können und gegebenenfalls durch 1 bis 4 zusätzliche (Meth)-Acryloyl-oxy-Reste substituiert sein können, bedeuten, und die Ringe A und B gleich oder verschieden sind und aromatisch oder gesättigt sein können.

Die (Meth)-Acrylsaure-Derivate können als reine Isomere oder als Isomerengemisch vorliegen. Für die erfindungsgemäße Verwendung der (Meth)-Acrylsäure-Derivate in Dentalmaterialien ist es besonders vorteilhaft, die Isomerengemische einzusetzen, da sie eine niedrigere Viskosität als die isomerenreinen Verbindungen besitzen.

Im Rahmen der vorliegenden Erfindung können die Substituenten im allgemeinen folgende Bedeutung haben.

In der Definition von $R^1$ und $R^2$ bedeuten Alkylreste mit 1-6 Kohlenstoffatomen, bevorzugt sind solche mit 1-4 Kohlenstoffatomen, beispielsweise Methyl, Ethyl, Propyl, Isopropyl, Isobutyl, Pentyl, Isopentyl, Hexyl und Isohexyl.

Zweiwertige Kohlenwasserstoffreste $Y^1$ bis $Y^3$ sind geradkettige oder verzweigte aliphatische Kohlenwasserstoffreste mit 2 bis 15 Kohlenstoffatomen, bevorzugt 2 bis 10 Kohlenstoffatomen. Die Reste $Y^1$ und $Y^3$ konnen gegebenenfalls 1 bis 3 Sauerstoffbrücken, bevorzugt 1 oder 2 Sauerstoffbrücken enthalten. Es ist auch möglich, daß die Reste $Y^1$ bis $Y^3$ durch 1 bis 4, bevorzugt 1 oder 2 (Meth)-Acryloyloxyreste, substituiert sind. Beispielsweise seien die folgenden Reste genannt:

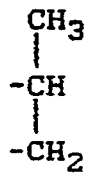 , 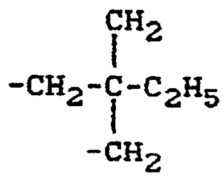 , 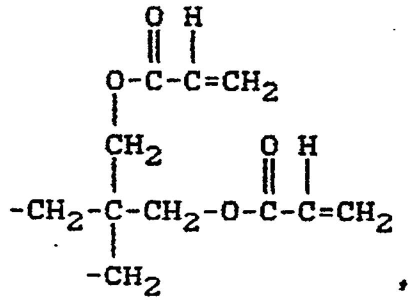 ,

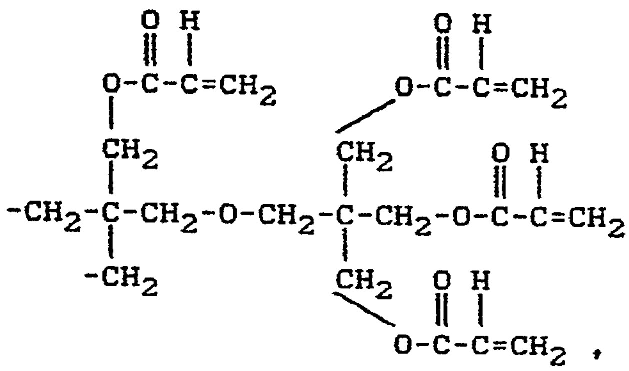 ,

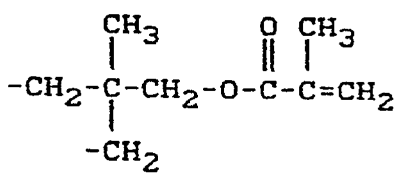 ,

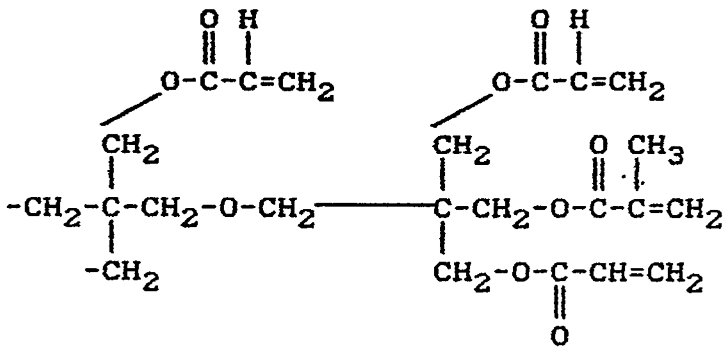

,

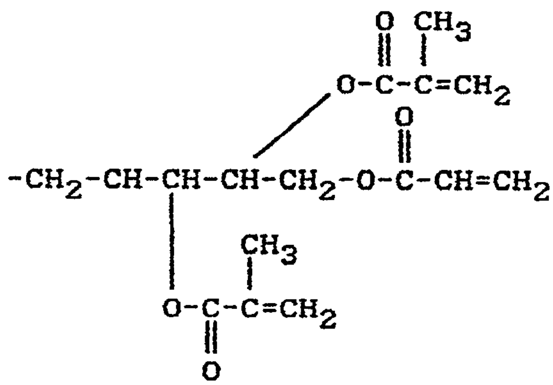

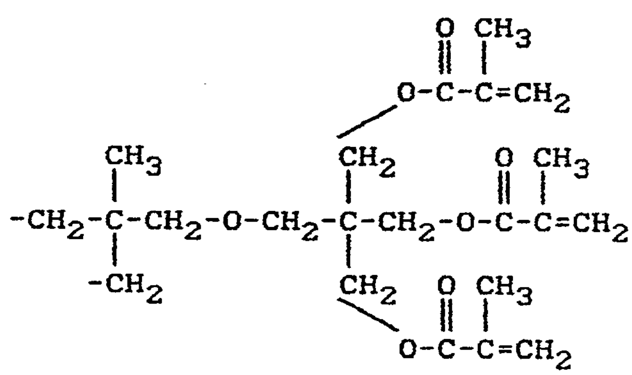

,

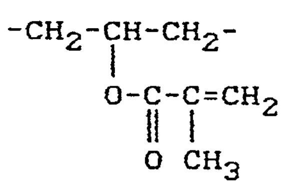

,

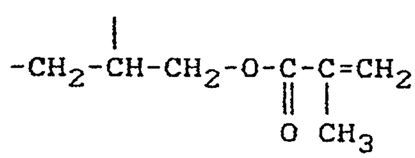

,

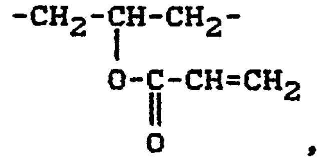 , 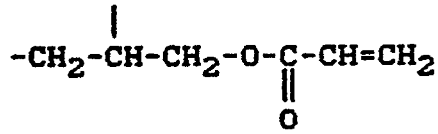 .

Der Ring A steht für einen Benzolkern oder einen Cyclohexanrest, der zwei, bzw. drei Substituenten enthält. Der Ring B steht für einen Benzolkern oder einen Cyclohexanrest, der drei bzw. vier Substituenten enthält.

Die neuen (Meth)-Acrylsäure-Derivate sind farblos, schwerflüchtig und ergeben nach der Polymerisation transparente Kunststoffe mit hoher Verschleißfestigkeit.

Sie lassen sich besonders gut in Dentalmaterialien, wie Zahnfüllmassen und Beschichtungsmitteln, verwenden. Die so erhaltenen Materialien zeichnen sich durch eine überraschend große Widerstandsfähigkeit gegenüber physikalischer und chemischer Beanspruchung aus. In besonderem Maße sind Härte und Bruchfestigkeit gegenüber üblichen, zu diesem Zweck eingesetzten Materialien, verbessert.

Bevorzugte (Meth)-Acrylsaure-Derivate sind Verbindungen der Formel

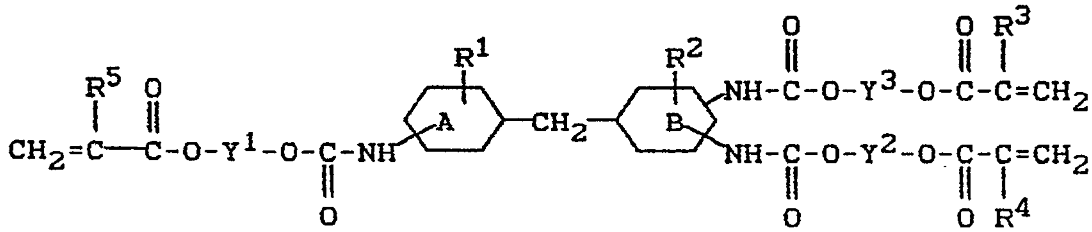

in der
$R^1$ für Wasserstoff steht,
$R^2$ für Wasserstoff oder einen Alkylrest mit 1 bis 4 Kohlenstoffatomen steht,
$R^3$, $R^4$ und $R^5$ gleich oder verschieden sind und Wasserstoff oder Methyl bedeuten,
$Y^1$ bis $Y^3$ gleich oder verschieden sind und zweiwertige geradkettige oder verzweigte aliphatische Kohlenwasserstoffreste mit 2 bis 10 Kohlenstoffatomen, die gegebenenfalls 1 oder 2 Sauerstoffbrücken enthalten können, und gegebenenfalls durch 1 bis 2 zusatzliche (Meth)-Acryloyloxyreste substituiert sein können, bedeuten, der Ring B aromatisch oder gesättigt ist und der Ring A gesättigt ist.

Insbesondere bevorzugt sind (Meth)-Acrylsäure-Derivate der Formel:

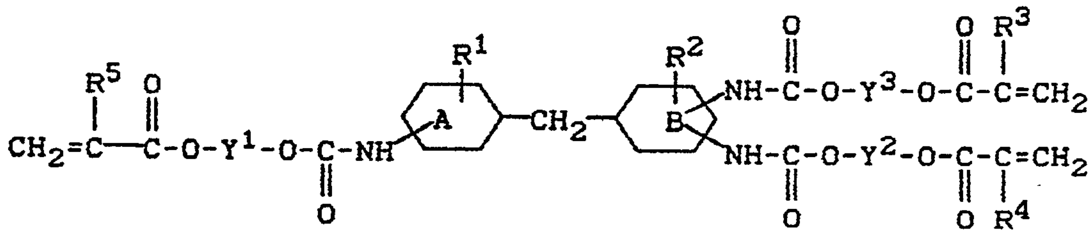

in der
$R^1$ für Wasserstoff steht,
$R^2$ für Wasserstoff oder Methyl steht,
$R^3$, $R^4$ und $R^5$ gleich oder verschieden sind und Wasserstoff oder Methyl bedeuten,
$Y^1$ bis $Y^3$ gleich oder verschieden sind und zweiwertige geradkettige oder verzweigte aliphatische Kohlenwasserstoffreste mit 2 bis 10 Kohlenstoffatomen, die gegebenenfalls 1 oder 2 Sauerstoffbrücken enthalten können und gegebenenfalls durch 1 oder 2 zusätzliche (Meth)-Acryloyloxyreste substituiert sein können, bedeuten und die Ringe A und B gesättigt sind.

Beispielsweise seien die folgenden (Meth)-Acrylsäure-Derivate genannt:

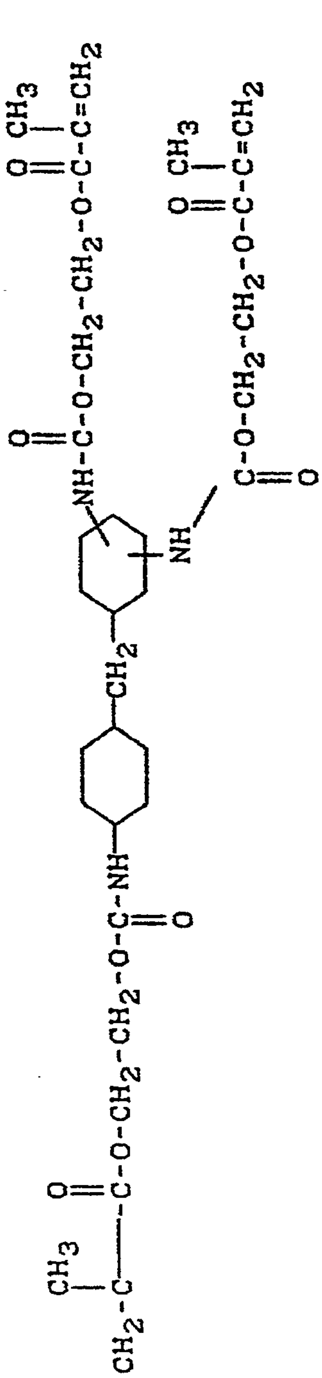

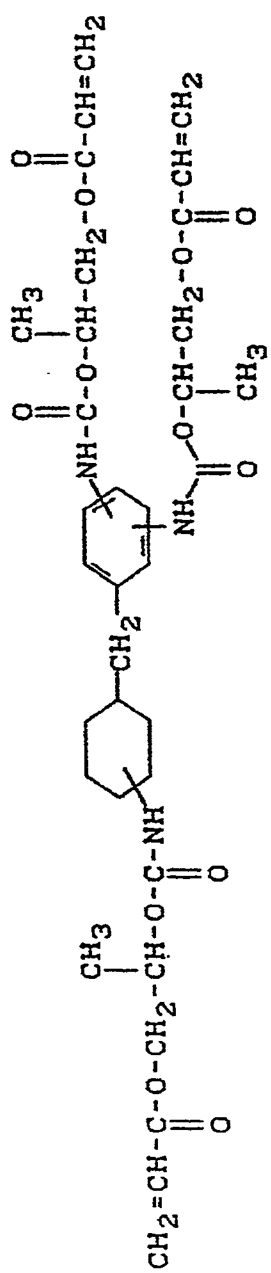

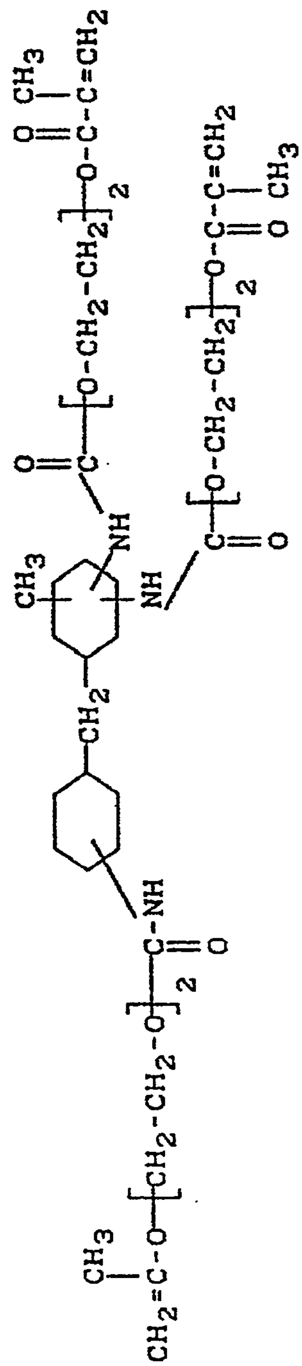

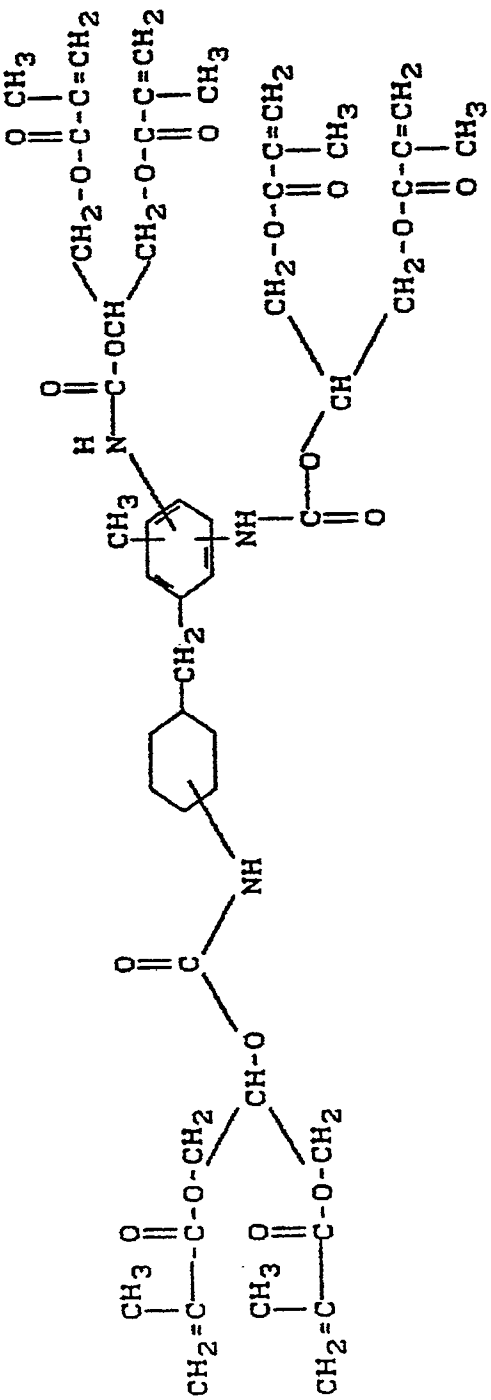

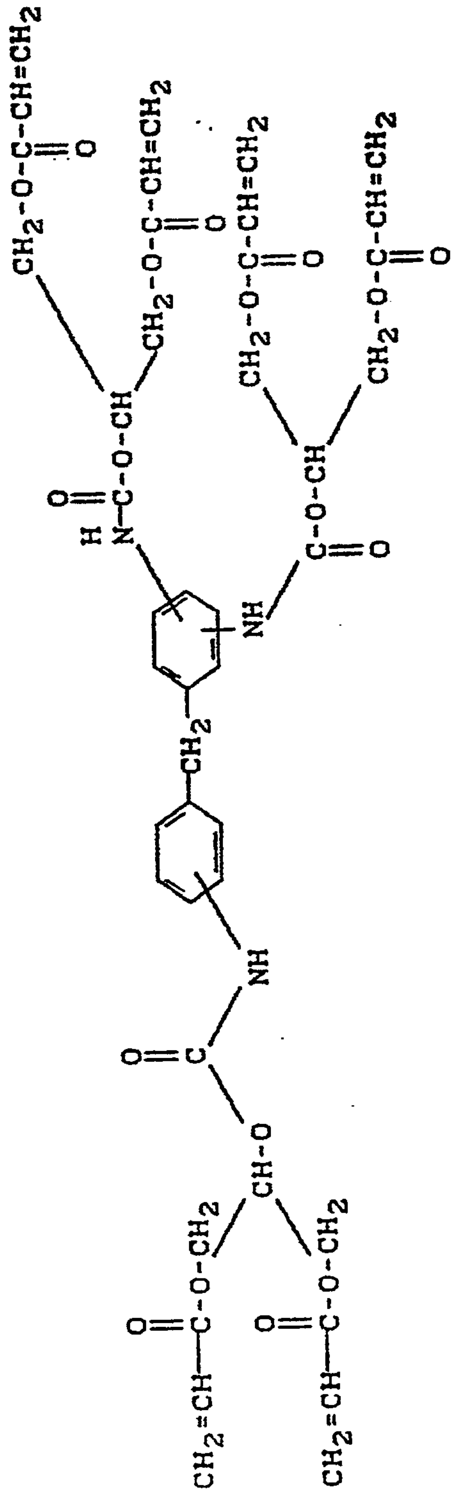

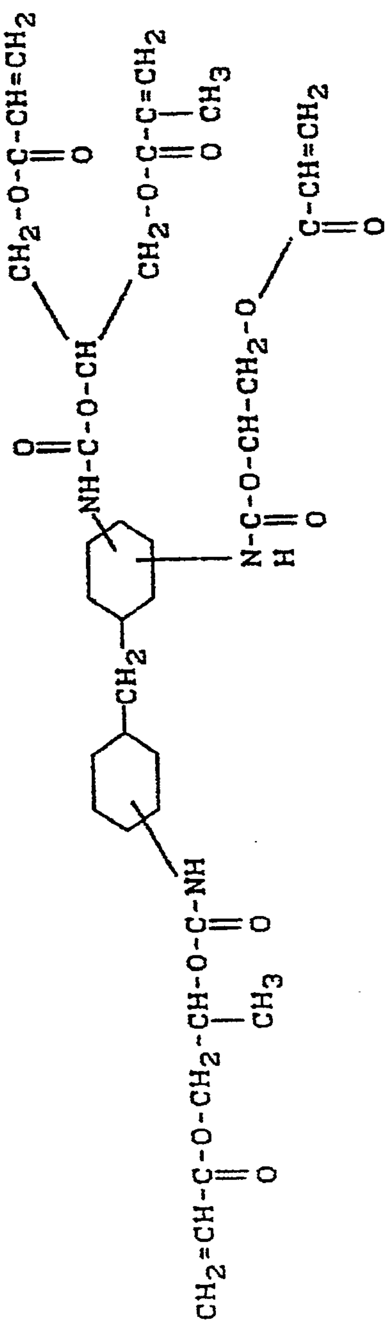

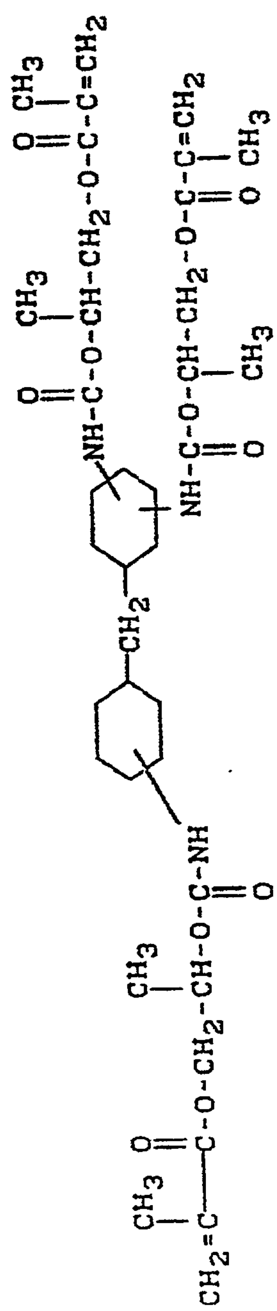

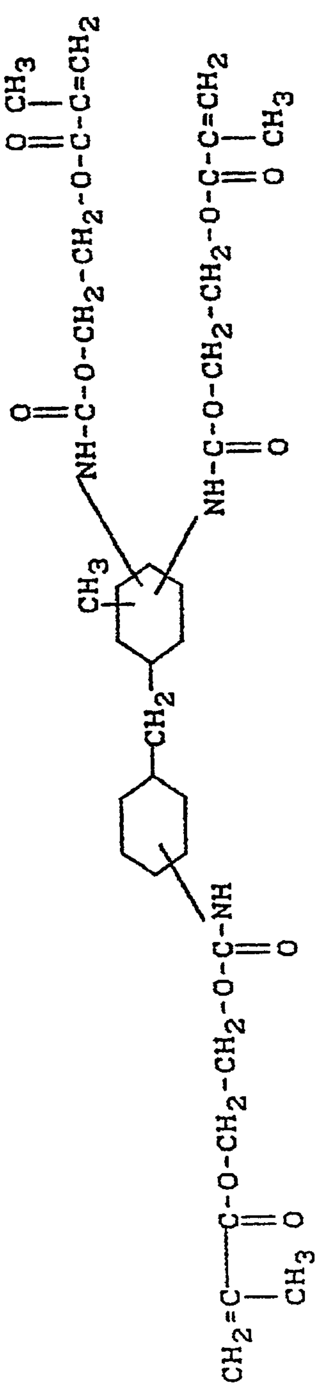

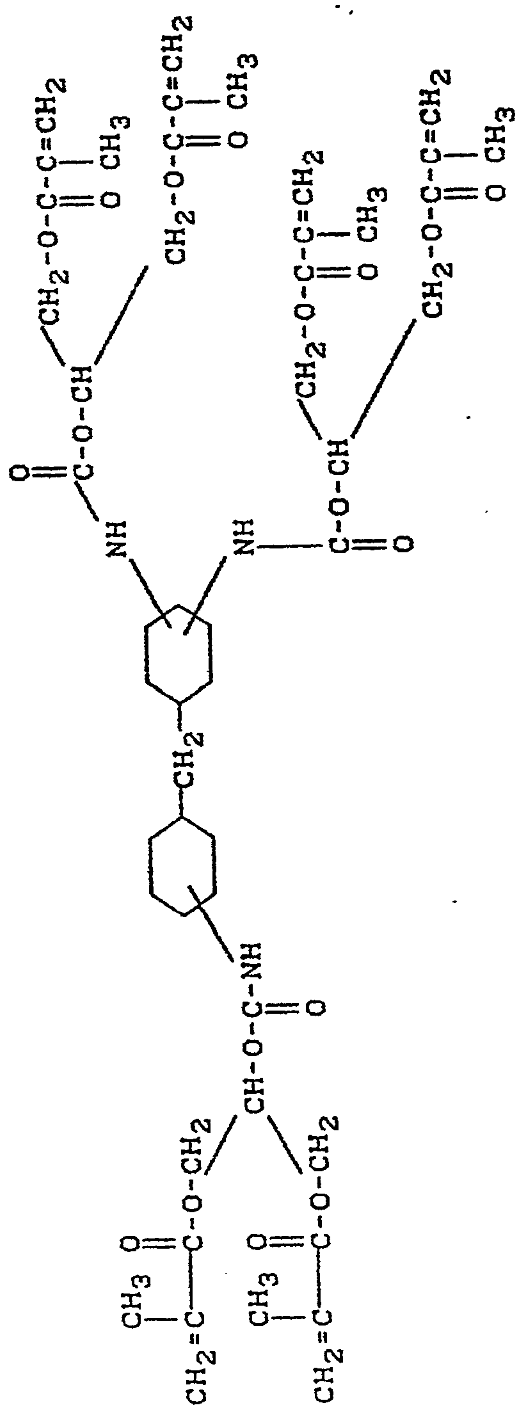

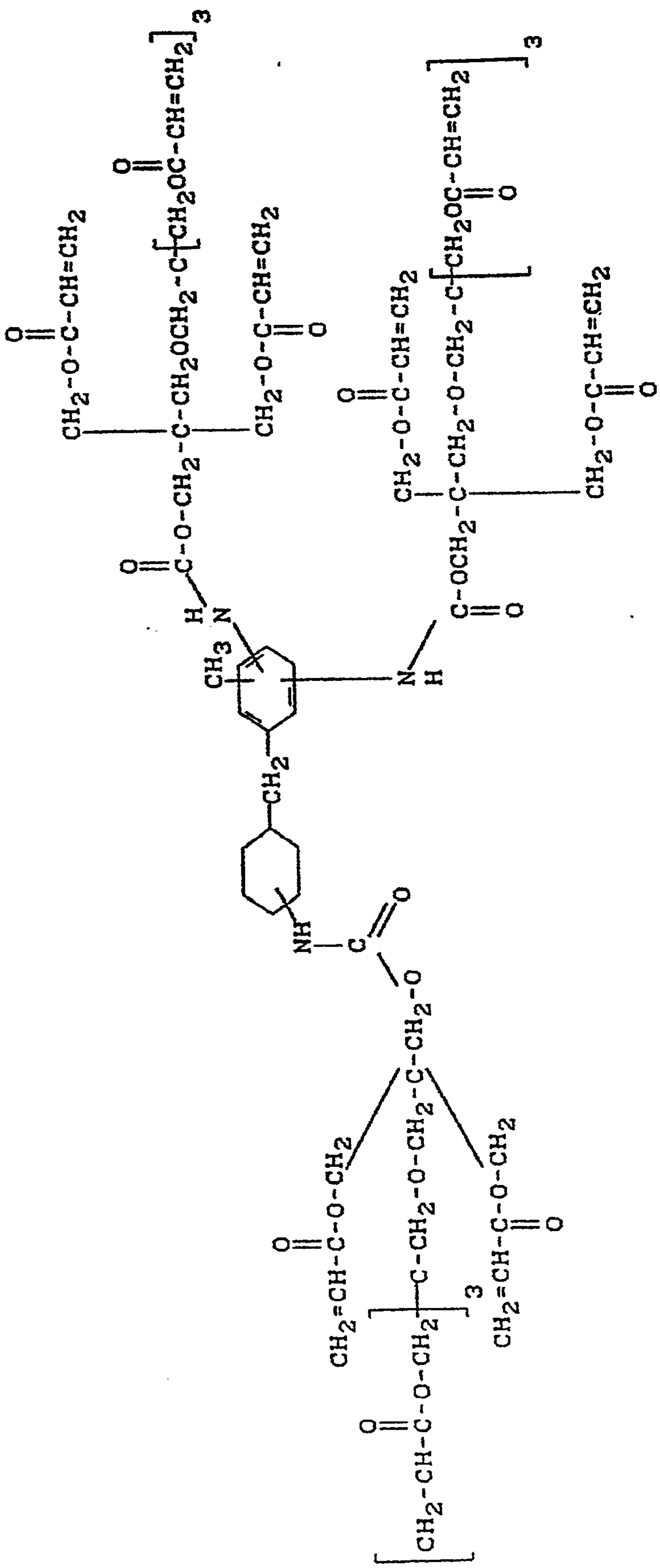

Es wurde auch ein Verfahren zur Herstellung dr erfindungsgemässen (Meth)-Acrylsäure-Derivate gefunden, das dadurch gekennzeichnet ist, daß man ein Triisocyanat der Formel

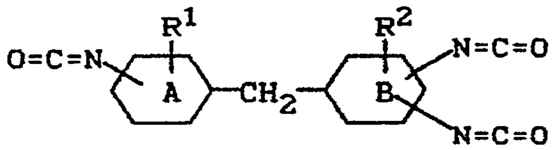

(II)

in der

$R^1$ und $R^2$ gleich oder verschieden sind und für Wasserstoff oder einen Alkylrest mit 1-6 Kohlenstoffatomen stehen und die Ringe A und B gleich oder verschieden sind und aromatisch oder gesättigt sein können,

mit einem Hydroxyalkyl-(Meth)-Acrylsäureester der Formel

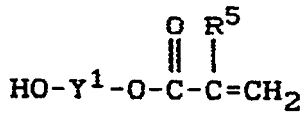

(III)

und/oder

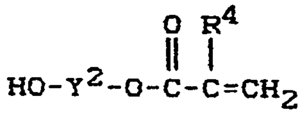

(IV)

und/oder

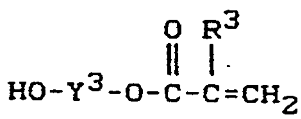

(V)

in der

$R^3$, $R^4$, $R^5$ und $R^6$ gleich oder verschieden sind und Wasserstoff oder Methyl bedeuten und

$Y^1$ bis $Y^3$ gleich oder verschieden sind und zweiwertige geradkettige oder verzweigte aliphatische Kohlenwasserstoffreste mit 2 bis 15 Kohlenstoffatomen, die gegebenenfalls 1 bis 3 Sauerstoffbrücken enthalten können, und gegebenenfalls durch 1 bis 4 zusätzliche (Meth)-Acryloyloxyreste substituiert sein können, bedeuten, umsetzt.

Triisocyanate der Formel II sind bekannt (DE-A1 3 417 684, DE-A1 3 417 683) und konnen durch Phosgenierung der entsprechenden Triaminoverbindungen erhalten werden.

Hydroxyalkyl-(Meth)-Acrylsäureester der Formel III bis V sind handelsüblich oder können in bekannter Weise durch partielle Veresterung der entsprechenden Polyole hergestellt werden.

Das erfindungsgemäße Verfahren wird im allgemeinen so durchgeführt, daß man, bezogen auf jede Isocyanatgruppe des Triisocyanats (II) 0,9 bis 1,1, bevorzugt 1,0 bis 1,05 Mol eines Hydroxyalkyl-(Meth)-Acrylsäureesters der Formel III, IV oder V oder Gemische dieser Verbindungen einsetzt, wobei die Summe aller Hydroxyl-Äquivalente, bezogen auf jede Isocyanatgruppe des Triisocyanats (II), 0,9 bis 1,1, bevorzugt 1,0 bis 1,05, ergeben soll.

Das erfindungsgemäße Verfahren wird im allgemeinen unter Wasserausschluß in einem inerten Lösungsmittel durchgeführt. Beispielsweise seien Chloroform, Tetrahydrofuran, Aceton, Dioxan, Dichlormethan, Toluol und Acetonitril genannt bevorzugte Lösungsmittel sind Chloroform, Toluol, Aceton und Dichlormethan.

Das erfindungsgemäße Verfahren wird im allgemeinen im Temperaturbereich von 20 bis 100°C, vorzugsweise von 30 bis 70°C, durchgeführt.

Das erfindungsgemäße Verfahren wird im allgemeinen bei Normaldruck durchgeführt. Es ist jedoch auch möglich, das Verfahren im Druckbereich von 1 bis 15 bar durchzuführen.

Bei der erfindungsgemäßen Umsetzung zum Urethan wird vorzugsweise unter Wasserausschluß gearbeitet (vorzugsweise unter 0,1% Wasser).

Zur Beschleunigung der Umsetzung werden vorzugsweise zinnhaltige Katalysatoren wie Dibutylzinndilaurat, Zinn(II)-octoat oder Dibutylzinndimethoxid verwendet.

Es ist auch möglich, als Katalysatoren Verbindungen mit tertiaren Aminogruppen oder Titanverbindungen einzusetzen. Beispielsweise seien die folgenden Katalysatoren genannt: Diazabicyclo[2.2.2]octan, Triethylamin, N-Methylpiperidin, Tetrabutoxy-titan (Ullmann, Encyclopädie der technischen Chemie, Bd. 19, S. 306 (1981)).

Im allgemeinen wird der Katalysator in einer Menge von 0,01 bis 2,5 Gew.-%, bevorzugt von 0,1 bis 1,5 Gew.-%, bezogen auf die Gesamtmenge der Reaktanden eingesetzt.

Die Umsetzung zu Urethan wird im allgemeinen in Gegenwart von 0,01 bis 0,2 Gew.-% eines Polymerisationsinhibitors, beispielsweise 2,6-Di-tert.-butyl-4-methylphenol durchgeführt,

Das erfindungsgemäße Verfahren kann beispielsweise wie folgt durchgeführt werden:

Die Reaktanden werden in dem Lösungsmittel gelöst und unter Rühren mit dem Katalysator versetzt. Der zeitliche Verlauf der Umsetzung kann beispielsweise durch Messung der IR-Spektren verfolgt werden. Nach vollständiger Umsetzung der Isocyanatgruppen werden die Reaktionsprodukte durch Entfernen des Lösungsmittels isoliert. Eine vorherige Reinigung mit Hilfe von Absorbentien, beispielsweise Aktivkohle, Bleicherde, Kieselgel oder Aluminiumoxid ist selbstverständlich auch möglich.

Die erfindungsgemäßen (Meth)-Acrylsäure-Derivate von Triisocyanaten können als Monomere zur Herstellung von polymeren Werkstoffen verwendet werden. Die Polymerisation kann in an sich bekannter Weise durch radikalische Initiierung durchgeführt werden und ergibt Polymerisate, die eine hohe Vernetzungsdichte aufweisen.

Die erfindungsgemäßen (Meth)-Acrylsäurederivate von Triisocyanaten können insbesondere als Monomere für Dentalmaterialien verwendet werden. Als Dentalmaterialien seien beispielsweise Füllungsmaterialien für Zähne, Beschichtungsmittel für Zähne und Komponenten für die Herstellung von Zahnersatz genannt. Je nach Anwendungsgebiet können Dentalmaterialien weitere Hilfsstoffe enthalten.

Für die Anwendung als Monomere für Zahnfüllmassen oder Beschichtungsmittel (Zahnlacke) im Dentalbereich können die erfindungsgemäßen (Meth)-Acrylsäure-Derivate von Triisocyanaten mit an sich bekannten Comonomeren gemischt werden. So kann beispielsweise die Viskosität dem Verwendungszweck angepaßt werden. Diese Monomermischungen haben im allgemeinen eine Viskosität im Bereich von 60 bis 10 000 mPa.s.

Beispielsweise seien die folgenden Comonomere genannt:

Triethylenglykoldimethacrylat, Tetraethylenglykoldimethacrylat, 1,12-Dodecandioldimethacrylat, 1,6-Hexandioldimethacrylat, Diethylenglykoldimethacrylat, 2,2-Bis[p(2'-hydroxy-3'methacryloyloxypropoxy) phenyl]propan, 2,2Bis[p-(2'-methacryloyloxyethoxy)phenyl]propan. Von Vorteil sind auch Comonomere mit Urethangruppen, z.B. die bekannten Umsetzungsprodukte von 1 Mol eines Diisocyanats, z.B. Hexamethylendiisocyanat, Trimethylhexamethylendiisocyanat, Isopharondiisocyanat, mit 2 Mol eines Hydroxyalkyl(meth)acrylsäureesters, z.B. Glycerindimethacrylat, 2-Hydroxypropylacrylat usw.

Weitere Beispiele für Comonomere sind:

Trimethylolpropan-tri(meth)-acrylat, Bis-(Meth)acryloyl-oxyethoxymethyl)-tricyclo[5.2.1.0.$^{2.6}$]decan (gemäß DE-A 2 931 925 und 2 931 926), 1,3-Di((meth)acryloyloxypropyl)-1,1,3,3-tetramethyl-disiloxan, 1,3-Bis(3-(meth)acryloyloxyethylcarbamoyloxy-propyl)-1,1,3,3-tetramethyl-disiloxan. Insbesondere werden Comonomere bevorzugt, die bei 13 mbar einen Siedepunkt über 100°C besitzen.

Im Rahmen der vorliegenden Erfindung ist es ebenfalls bevorzugt, Mischungen verschiedener erfindungsgemäßer (Meth)-Acrylsäure-Derivate einzusetzen.

Der Anteil der erfindungsgemäßen (Meth)Acrylsäure-Derivate von Triisocyanaten in den Monomermischungen beträgt im allgemeinen 10 bis 90 Gew.%, vorzugsweise 20 bis 75 Gew.-%.

Es ist auch möglich, Monomermischungen einzusetzen, die mehrere Comonomere enthalten.

Die erfindungsgemäßen (Methl)-Acrylsäure-Derivate von Triisocyanaten lassen sich, gegebenenfalls in Mischung mit den genannten Monomeren, mit an sich bekannten Methoden zu vernetzten Polymerisaten aushärten (G.M. Brauer, H. Argentar, Am. Chem. Soc., Symp. Ser. 212, S. 359-371 (1983). Für die sogenannte Redoxpolymerisation ist ein System aus einer peroxidischen Verbindung und einem Reduktionsmittel, beispielsweise auf Basis tertiärer aromatischer Amine, geeignet. Beispiele für Peroxide sind:

Dibenzoylperoxid, Dilauroylperoxid und Di-4-Chlorbenzoylperoxid.

Als tertiäre aromatische Amine seien beispielsweise N,N-Dimethyl-p-toluidin, Bis-(2-hydroxyethyl)-p-toluidin, Bis(2-hydroxyethyl)-3,5-dimethylanilin und das in der DE-A 2 759 239 beschriebene N-Methyl-N-(2-methyl-carbamoyloxypropyl)-3,5-dimethylanilin genannt.

Die Konzentration des Peroxids bzw. des Amins wird vorteilhaft so gewählt, daß sie 0,1 bis 5 Gew.-%, bevorzugt 0,5 bis 3 Gew.-%, bezogen auf die Monomermischung beträgt. Die Peroxid- bzw. Aminhaltige Monomermischungen werden bis zur Anwendung getrennt gelagert.

Die erfindungsgemäßen Monomeren können auch durch Bestrahlung mit UV-Licht oder sichtbarem Licht (beispielsweise im Wellenlängenbereich von 230 bis 650 nm) zur Polymerisation gebracht werden. Als Initiatoren für die fotoinitiierte Polymerisation eignen sich beispielsweise Benzil, Benzildimethylketal, Benzoinmonoalkylether, Benzophenon, p-Methoxybenzophenon, Fluorenon, Thioxanthon, Phenanthrenchinon und 2,3-Bornandion (Campherchinon), gegebenenfalls in Gegenwart von synergistisch wirkenden Fotoaktivatoren, wie N,N-Dimethylaminoethylmethacrylat, Triethanolamin, 4-N,N-Dimethyl-aminobenzolsulfonsäurebisallylamid.

Die Durchführung des Fotopolymerisationsverfahrens ist beispielsweise in der DE-A 3 135 115 beschrieben.

Neben den oben beschriebenen Initiatoren können den erfindungsgemäßen (Meth)-Acrylsäureestern an sich für diesen Einsatzzweck bekannte Lichtschutzmittel und Polymerisations-Inhibitoren zugesetzt werden.

Das Lichtschutzmittel und der Polymerisations-Inhibitor werden jeweils im allgemeinen in einer Menge von 0,01 bis 0,50 Gew.-Teilen, bezogen auf 100 Gew.-Teile der Monomermischung eingesetzt. Die Monomermischungen können ohne Zusatz von Füllstoffen als Beschichtungsmittel für Zähne (Zahnlacke) eingesetzt werden. Nach der Polymerisation erhält man einen kratzfesten Überzug auf dem Substrat.

Bei der Verwendung als Zahnfüllmassen setzt man den erhaltenen Monomermischungen im allgemeinen Füllstoffe zu. Um einen hohen Füllgrad erreichen zu können, sind Monomermischungen, die eine Viskosität im Bereich von 60 bis 10 000 mPa.s besitzen, besonders vorteilhaft. Den die erfindungsgemäßen Verbindungen der Formel I enthaltenden Monomermischungen können vorzugsweise anorganische Füllstoffe zugemischt werden. Beispielsweise seien Bergkristall, Quarzit, Kristobalit, Quarzglas, hochdisperse Kieselsäure, Aluminiumoxid und Glaskeramiken, beispielsweise Lanthan und Zirkon enthaltende Glaskeramiken (DE-A 2 347 591) genannt.

Die anorganischen Füllstoffe werden zur Verbesserung des Verbundes zur Polymermatrix des Polymethacrylats vorzugsweise mit einem Haftvermittler vorbehandelt. Die Haftvermittlung kann beispielsweise durch eine Behandlung mit Organosiliciumverbindungen erreicht werden (E.P. Plueddemann, Progress in Organic coatings, 11, 297 bis 308 (1983)). Bevorzugt wird 3-Methacryloyloxypropyltrimethoxysilan eingesetzt.

Die Füllstoffe für die erfindungsgemäßen Zahnfüllmassen weisen im allgemeinen einen mittleren Teilchendurchmesser von 0,01 bis 100 µm, vorzugsweise von 0,05 bis 50 µm auf, besonders bevorzugt 0,05 bis 5 µm. Es kann auch vorteilhaft sein, mehrere Füllstoffe nebeneinander einzusetzen, die einen voneinander verschiedenen Teilchendurchmesser und verschiedenen Silanisierungsgrad besitzen.

Der Füllstoffanteil in den Zahnfüllmassen beträgt im allgemeinen 5 bis 85 Gew.-%, vorzugsweise 50 bis 80 Gew.-%.

Für die Herstellung der Zahnfüllmassen werden die Komponenten unter Verwendung handelsüblicher Knetmaschinen verarbeitet,

Der Anteil der erfindungsgemäßen (Meth)-Acrylsäure-Derivate in den Füllmassen beträgt im allgemeinen 5 bis 90 Gew.-%, vorzugsweise 10 bis 60 Gew.-%, bezogen auf die Füllmasse. Die Aushärtung der Zahnfüllmassen zu einem Formkorper erfolgt in der Kavität des Zahnes mit den oben genannten Methoden. Aufgrund der hohen Verschleißfestigkeit der erhaltenen Zahn-Füllung sind Zahnfüllmassen, die die erfindungsgemäßen Verbindungen in polymerisierter Form enthalten, insbesondere zur Anwendung im Seitenzahnbereich geeignet.

Die erfindungsgemäßen (Meth)-Acrylsäure-Derivate von Triisocyanaten können auch als Komponenten bei der Herstellung von Zahnersatz eingesetzt werden.

Dabei werden die erfindungsgemäßen Monomeren mit den üblicherweise verwendeten, an sich bekannten Bestandteilen kombiniert. Vorzugsweise werden die Monomeren im Gemisch mit Alkylmethacrylatem, wie Methylmethacrylat eingesetzt. Es können auch zusätzlich an sich bekannte Perlpolymerisate zugesetzt werden. Zur Einstellung der Zahnfarbe konnen bekannte anorganische und organische Farbpigmente und Trübungsmittel zugesetzt werden. Auch die Anwendung von Stabilisatoren und Lichtschutzmitteln ist möglich.

Die Kunststoffzähne werden durch radikalische Polymerisation der Dentalmassen unter Formgebung hergestellt. Die Verarbeitung ist sowohl nach Injektionsverfahren als auch Prägeverfahren möglich und erfolgt im allgemeinen nach den üblichen Herstellungsmethoden für Zähne auf Basis von Poly(methylmethacrylat), z.B. durch thermische Polymerisation unter Verwendung von an sich bekannten Polymerisationsinitiatoren, beispielsweise auf Basis von Peroxiden und Azoverbindungen, wie Dibenzoylperoxid, Dilauroylperoxid, Cyclohexylpercarbonat, Azoisobuttersuäredinitril. Gut geeignet sind auch Mischungen von Polymerisationsinitiatoren mit unterschiedlichen Zerfallstemperaturen.

Beispiel 1

Herstellung des Adduktes aus Dicyclohexylmethantriisocyanat und Glycerindimethacrylat

45,45 g (0,15 mol) Dicyclohexylmethan-triisocyanat (Isomerengemisch mit einem NCO-Gehalt von 41,5 Gew.-%) werden in 100 ml Chloroform gelöst. Diese Mischung wird mit 57 mg 2,6-Di-tert.-butyl-4-methylphenol und 100 mg Zinn-(II)-oktoat versetzt. Bei 30°C werden unter Rühren 102,6 g (0,45 mol) Glycerinclimethacrylat (Isomerengemisch von 1,3- und 1,2-Bis-methacryloyloxy-propanol) zugetropft. Nach beendeter Zugabe wird bei 50 – 60°C gerührt, bis die Isocyanatgruppen vollständig umgesetzt sind.

Der Umsatz wird durch Messung der IR-Spektren (Isocyanatbande bei ~ 2200 $cm^{-1}$) verfolgt.

Das Reaktionsgemisch wird abgekühlt, mit Aktivkohle verrührt, über Celite® filtriert und im Wasserstrahlvakuum vom Lösungsmittel befreit.

Der Rückstand wird im Hochvakuum bis zur Gewichtskonstanz eingeengt.

IR (Film auf KBr) [$cm^{-1}$]:
υ (N-H): 3 400
υ (C=O): 1690 – 1750 (Ester und Amid I)
υ (C=O): 1000 – 1530 (Amid II)
υ (C=C): 1638 $cm^{-1}$

Beispiel 2

Die Herstellung des Adduktes von Triisocyanato-Dicyclohexylmethan und Glycerindimethacrylat wird, wie in Beispiel 1 beschrieben, durchgeführt. Nach beendeter Umsetzung wird jedoch das Reaktionsgemisch abgekühlt, mit Aktivkohle verrührt, über Celite® filtriert und mit 95,95 g Triethylenglykoldimethacrylat versetzt. Die Mischung wird im Vakuum bis zur Gewichtskonstanz eingeengt. Man erhält ein farbloses Monomergemisch, das direkt als Monomerlösung für die Herstellung von Dentalmaterialien eingesetzt werden kann. Der Gehalt des erfindungsgemäßen (Meth)-Acrylsäure-Derivates beträgt 60, 7 Gew.-%.

Beispiel 3

Herstellung des Adduktes von Triisocyanato-methyldicyclohexylmethan und Glycerindimethacrylat 38,9 g (0,1227 Mol) Triisocyanato-methyldicyclohexylmethan (Isomerengemisch mit einem NCO-Gehalt von 37 Gew.-%) werden in 200 ml Chloroform gelöst und mit 53,7 mg 2,6-Di-tert.butyl-4-methylphenol und 100 mg Dibutylzinndilaurat versetzt. Bei 50°C werden unter Rühren 83,9 g (0,368 Mol) Glycerindimethacrylat (Isomerengemisch von 1,3- und 1,2-Bismethacryloyloxypropanol) langsam zugetropft. Man rührt bis zum vollständigen Umsatz (IR-Kontrolle) der Isocyanatgruppen bei 50 – 60°C. Das Produkt wird, wie in Beispiel 1 beschrieben, isoliert. Das erhaltene Trisurethanhexanmethacrylat ist bei Raumtemperatur nicht mehr gießbar.

Die Verdünnung mit Triethylenglykoldimethacrylat auf einen Gehalt von 65 Gew.-% des erfindungsgemäßen (Meth)Acrylsäure-Derivates liefert ein gebrauchsfertiges Monomergemisch mit einer Viskosität von etwa 1 Pa.s.

Beispiel 4

Herstellung des Adduktes au, Triisocyanatodicyclohexylmethan mit 2-Hydroxypropylacrylat 30,3 g (0,1 Mol) Triisocyanato-Dicyclohexylmethan (NCO-Gehalt 41,5 Gew.-%), 35 mg 2,6-Di-t-Butyl-4-methylphenol und 0,1 g Zinn(II)-oktoat werden in 100 ml Chloroform gelöst. Bei Raumtemperatur werden 39 g (0,3 Mol) 2-Hydroxypropylacrylat zugetropft. Man rührt bei 60°C bis zum vollständigen Umsatz der NCO-Gruppen. Das Reaktionsgemisch wird analog zu Beispiel 1 aufgearbeitet. IR (Film auf KBr) [$cm^{-1}$]:
υ (N-H): 3 400, υ (C=O) 1690 – 1760,
υ (C=O) (Amid II): 1500, υ (C=C): 1620, 1640

Beispiel 5

Herstellung des Adduktes aus Triisocyanato-dicyclohexylmethan und 2-Hydroxypropylmethacrylat 30,3 g (0,1 Mol) Triisocyanato-dicyclohexylmethan (NCOGehalt 41,5 Gew.-%), 37 mg 2,6-Di-tert.-butyl-4-methylphenol und 0,1 g Zinn-(II)-oktoat werden in 100 ml Chloroform mit 43,2 g (0,3 Mol) 2-Hydroxypropylmethacrylat bei 60°C umgesetzt. Die Produktisolierung erfolgt analog zu Beispiel 1.

Beispiel 6

Herstellung einer Zahnfüllmasse 198,3 Gewichtsteile einer Monomermischung aus Beispiel 2, bestehend aus 60,7 Gew.-% des Adduktes aus Triisocyanato-dicyclohexylmethan und Glycerindimethacrylat und 39,3 Gew.-% Triethylenglykoldimethacrylat, 0,4 Gewichtsteil Campherchinon, 0,25 Gewichtsteil Benzildimethylketal und 1,0 Gewichtsteil 4-N,N-Dimethylaminobenzosulfonsäurebisallylamid werden unter Lichtausschluß zu einer Monomerlösung verarbeitet. Diese härtet durch sichtbares Licht und/oder UV-Licht bei einer Belichtungsdauer von 60 Sekunden zu einem Kunststoff aus, der eine hohe mechanische Stabilität aufweist.

Zur Herstellung einer Zahnfüllmasse werden 38 Gewichtsteile der oben genannten Monomerlösung und 62 Gewichtsteile einer mit 5 Gew.-% 3-Methacryloyloxypropyl-trimethoxysilan silanisierten pyrogenen Kieselsäure (Aerosil OX 50) in einem handelsüblichen Kneter bei Raumtemperatur zu einer Paste verarbeitet. Ein nach DIN 13 922 mit einer handelsüblichen Dentallampe (Translux®) ausgehärteter Probekörper, der aus dieser Paste hergestellt wurde, zeigt einen sehr hohen Biegemodul, eine sehr hohe Biegefestigkeit und eine verbesserte Abrasionsbeständigkeit.

**Patentansprüche**

1. (Meth)-Acrylsäure-Derivate von Triisocyanaten der Formel

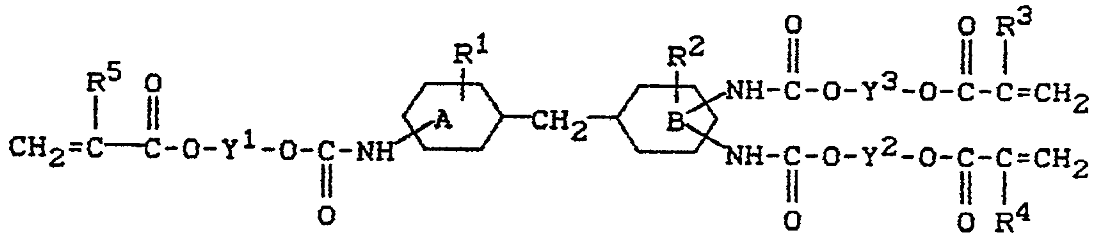

in der
$R^1$ und $R^2$ gleich oder verschieden sind und für Wasserstoff oder einen Alkylrest mit 1-6 Kohlenstoffatomen stehen, $R^3$, $R^4$ und $R^5$ gleich oder verschieden sind und Wasserstoff oder Methyl bedeuten,
$Y^1$ bis $Y^3$ gleich oder verschieden sind und zweiwertige geradkettige oder verzweigte aliphatische Kohlenwasserstoffreste mit 2 bis 15 Kohlenstoffatomen, die gegebenenfalls 1 bis 3 Sauerstoffbrücken enthalten können und gegebenenfalls durch 1 bis 4 zusätzliche (Meth)-Acryloyloxy-Reste substituiert sein können, bedeuten, und die Ringe
A und B gleich oder verschieden sind und aromatisch oder gesättigt sein können.

2. (Meth)-Acrylsaure-Derivate von Triisocyanaten nach Anspruch 1, wobei
$R^1$ für Wasserstoff steht,
$R^2$ für Wasserstoff oder einen Alkylrest mit 1 bis 4 Kohlenstoffatomen steht,
$R^3$, $R^4$ und $R^5$ gleich oder verschieden sind und Wasserstoff oder Methyl bedeuten,
$Y^1$ bis $Y^3$ gleich oder verschieden sind und zweiwertige geradkettige oder verzweigte aliphatische Kohlenwasserstoffreste mit 2 bis 10 Kohlenstoffatomen, die gegebenenfalls 1 oder 2 Sauerstoffbrücken enthalten können und gegebenenfalls durch 1 oder 2 zusätzliche (Meth)Acryloyloxy-Reste substituiert sein können, bedeuten,
der Ring B aromatisch oder gesättigt ist und der Ring A gesättigt ist.

3. (Meth)-Acrylsaure-Derivate von Triisocyanaten nach Ansprüchen 1 und 2, wobei
$R^1$ für Wasserstoff steht,
$R^2$ für Wasserstoff oder Methyl steht,
$R^3$, $R^4$ und $R^5$ gleich oder verschieden sind und Wasserstoff oder Methyl bedeuten,
$Y^1$ bis $Y^3$ gleich oder verschieden sind und zweiwertige geradkettige oder verzweigte aliphatische Kohlenwasserstoffreste mit 2 bis 10 Kohlenstoffatomen, die gegebenenfalls 1 oder 2 Sauerstoffbrücken enthalten können und gegebenenfalls durch 1 oder 2 zusätzliche (Meth)-Acryloyloxy-Reste substituiert sein können, bedeuten, und die Ringe A und B gesättigt sind.

4. Ein Verfahren zur Herstellung der (Meth)-Acrylsäure-Derivate nach Ansprüchen 1 bis 3, dadurch gekennzeichnet, daß man ein Triisocyanat der Formel

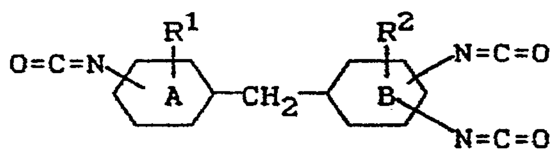

(II)

in der
$R^1$ und $R^2$ gleich oder verschieden sind und für Wasserstoff oder einen Alkylrest mit 1-6 Kohlenstoffatomen stehen und die Ringe A und B gleich oder verschieden sind und aromatisch oder gesättigt sein können,
mit einem Hydroxyalkyl-(Meth)-Acrylsäureester der Formel

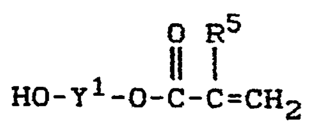

(III)

und/oder

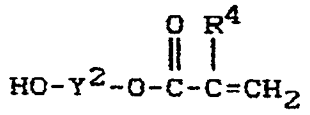 (IV)

und/oder

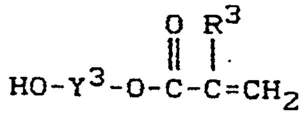 (V)

in der
$R^3$, $R^4$ und $R^5$ gleich cder verschieden sind und Wasserstoff oder Methyl bedeuten und
$Y^1$ bis $Y^3$ gleich oder verschieden sind und zweiwertige geradkettige oder verzweigte aliphatische Kohlenwasserstoffreste mit 2 bis 15 Kohlenstoffatomen, die gegebenenfalls 1 bis 3 Sauerstoffbrücken enthalten können und gegebenenfalls durch 1 bis 4 zusätzliche (Meth)-Acryloyloxy-Reste substituiert sein können, bedeuten, umsetzt.

5. Polymerisat aus (Meth)-Acrylsäure-Derivaten von Triisocyanaten der Formel

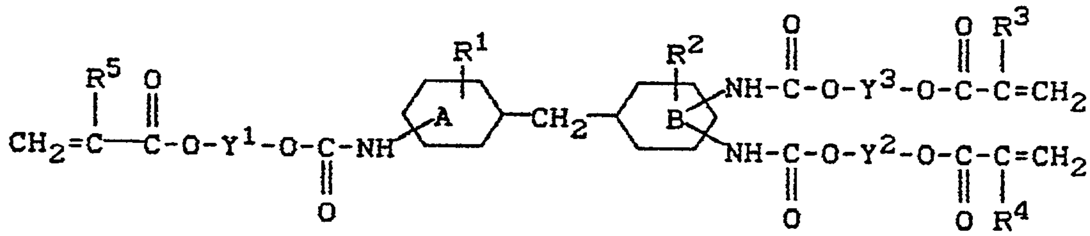

in der
$R^1$ und $R^2$ gleich oder verschieden sind und Wasserstoff oder Methyl bedeuten,
$Y^1$ bis $Y^3$ gleich oder verschieden sind und zweiwertige geradkettige oder verzweigte aliphatische Kohlenwasserstoffreste mit 2 bis 15 Kohlenstoffatomen, die gegebenenfalls 1 bis 3 Sauerstoffbrücken enthalten können und gegebenenfalls durch 1 bis 4 zusätzliche (Meth)-Acryloyloxy-Reste substituiert sein können, bedeuten, und die Ringe A und B gleich oder verschieden sind und aromatisch oder gesättigt sein können.

6. Verwendung von (Meth)-Acrylsäure-Derivaten von Triisocyanaten der Formel

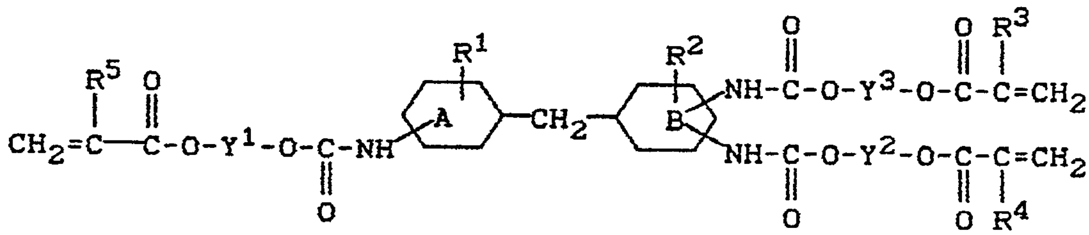

in der
$R^1$ und $R^2$ gleich oder verschieden sind und für Wasserstoff oder einen Alkylrest mit 1-6 Kohlenstoffatomen stehen,
$R^3$, $R^4$ und $R^5$ gleich oder verschieden sind und Wasserstoff oder Methyl bedeuten,
$Y^1$ bis $Y^3$ gleich oder verschieden sind und zweiwertige geradkettige oder verzweigte aliphatische Kohlenwasserstoffreste mit 2 bis 15 Kohlenstoffatomen, die gegebenenfalls 1 bis 3 Sauerstoffbrücken enthalten können und gegebenenfalls durch 1 bis 4 zusätzliche (Meth)-Acryloyloxy-Reste substituiert sein können, bedeuten, und die Ringe
A und B gleich oder verschieden sind und aromatisch oder gesättigt sein können, in zahntechnischen Werkstoffen.

7. Verwendung nach Anspruch 6, dadurch gekennzeichnet, daß die (Meth)-Acrylsäure-Derivate von Triisocyanaten in Zahnfüllmassen eingesetzt werden.

8. Verwendung nach Anspruch 6, dadurch gekennzeichnet, daß die (Meth)-Acrylsäure-Derivate von Triisocyanaten in Beschichtungsmitteln für Zähne eingesetzt werden.

9. Verwendung nach Anspruch 6, dadurch gekennzeichnet, daß die (Meth)-Acrylsäure-Derivate von Triisocyanaten für die Herstellung von Kunststoffzähnen eingesetzt werden.

10. Dentalmaterialien, dadurch gekennzeichnet, daß sie (Meth)-Acrylsäure-Derivate von Triisocyanaten der Formel

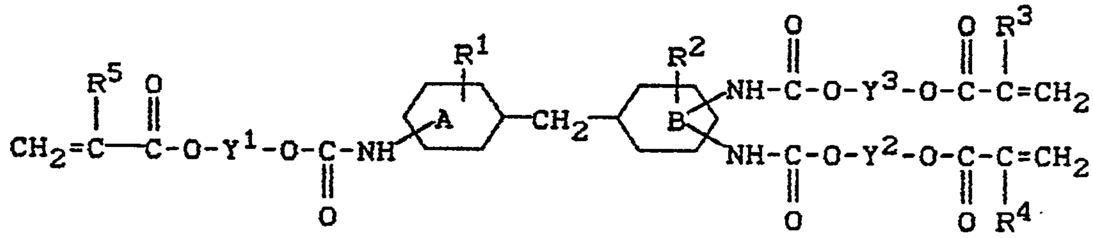

in der

$R^1$ und $R^2$ gleich oder verschieden sind und für Wasserstoff oder einen Alkylrest mit 1-6 Kohlenstoffatomen stehen,

$R^3$, $R^4$ und $R^5$ gleich oder verschieden sind und Wasserstoff oder Methyl bedeuten,

$Y^1$ bis $Y^3$ gleich oder verschieden sind und zweiwertige geradkettige oder verzweigte aliphatische Kohlenwasserstoffreste mit 2 bis 15 Kohlenstoffatomen, die gegebenenfalls 1 bis 3 Sauerstoffbrücken enthalten könnnen und gegebenenfalls durch 1 bis 4 zusätzliche (Meth)Acryloyloxy-Reste substituiert sein können, bedeuten, und

die Ringe A und B gleich oder verschieden sind und aromatisch oder gesättigt sein können, enthalten.

## Claims

1. (Meth)-acrylic acid derivatives of triisocyanates, of the formula

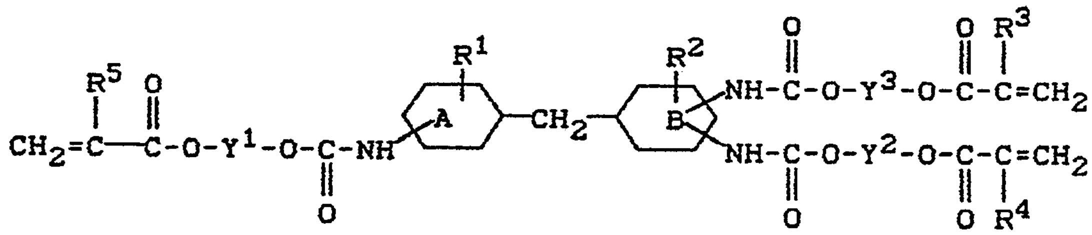

in which

$R^1$ and $R^2$ are identical or different and represent hydrogen or an alkyl radical having 1-6 carbon atoms,

$R^3$, $R^4$ and $R^5$ are identical or different and denote hydrogen or methyl,

$Y^1$ to $Y^3$ are identical or different and denote divalent straight-chain or branched aliphatic hydrocarbon radicals which have 2 to 15 carbon atoms, may optionally contain 1 to 3 oxygen bridges and can optionally be substituted by 1 to 4 additional (meth)-acryloyloxy radicals, and the rings A and B are identical or different and can be aromatic or saturated.

2. (Meth)-acrylic acid derivatives of triisocyanates, according to Claim 1, where

$R^1$ represents hydrogen,

$R^2$ represents hydrogen or an alkyl radical having 1 to 4 carbon atoms,

$R^3$, $R^4$ and $R^5$ are identical or different and denote hydrogen or methyl,

$Y^1$ to $Y^3$ are identical or different and denote divalent straight-chain or branched aliphatic hydrocarbon radicals which have 2 to 10 carbon atoms, may optionally contain 1 or 2 oxygen bridges and can optionally be substituted by 1 or 2 additional (meth)-acryloyloxy radicals, ring B is aromatic or saturated and ring A is saturated.

3. (Meth)-acrylic acid derivatives of triisocyanates, according to Claims 1 and 2, where

$R^1$ represents hydrogen,

$R^2$ represents hydrogen or methyl,

$R^3$, $R^4$ and $R^5$ are identical or different and denote hydrogen or methyl,

$Y^1$ to $Y^3$ are identical or different and denote divalent straight-chain or branched aliphatic hydrocarbon radicals which have 2 to 10 carbon atoms, may optionally contain 1 or 2 oxygen bridges and can optionally be substituted by 1 or 2 additional (meth)-acryloyloxy radicals, and rings A and B are saturated.

4. A process for the preparation of the (meth)acrylic acid derivatives according to Claims 1 to 3, characterized in that a triisocyanate of the formula

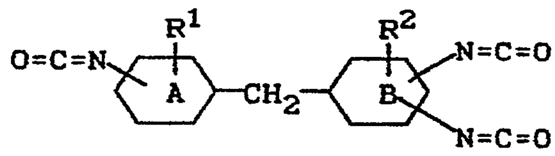 (II)

in which
$R^1$ and $R^2$ are identical or different and represent hydrogen or an alkyl radical having 1-6 carbon atoms, and rings A and B are identical or different and can be aromatic or saturated, is reacted with a hydroxyalkyl (meth)-acrylate of the formula

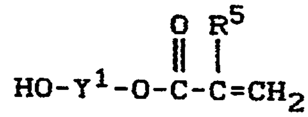 (III)

and/or

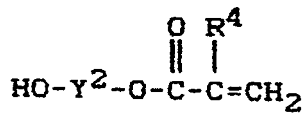 (IV)

and/or

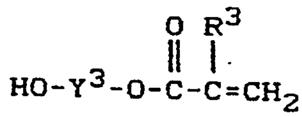 (V)

in which
$R^3$, $R^4$ and $R^5$ are identical or different and denote hydrogen or methyl, and
$Y^1$ to $Y^3$ are identical or different and denote divalent straight-chain or branched aliphatic hydrocarbon radicals which have 2 to 15 carbon atoms, may optionally contain 1 to 3 oxygen bridges and can optionally be substituted by 1 to 4 additional (meth)-acryloyloxy radicals.

5. Polymer made from (meth)-acrylic acid derivatives of triisocyanates, of the folmula

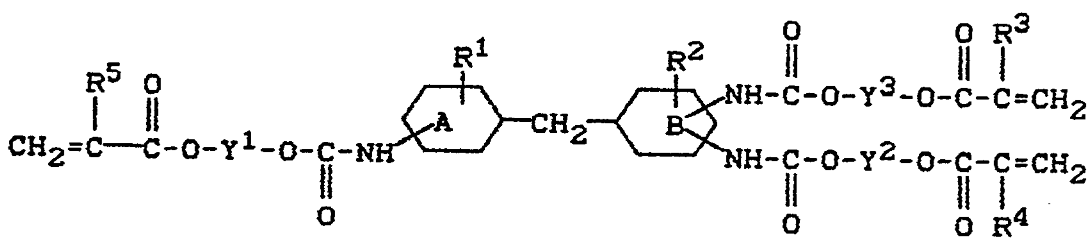

in which
$R^1$ and $R^2$ are identical or different and denote hydrogen or methyl,
$Y^1$ to $Y^3$ are identical or different and denote divalent straight-chain or branched aliphatic hydrocarbon radicals which have 2 to 15 carbon atoms, may optionally contain 1 to 3 oxygen bridges and can optionally be substituted by 1 to 4 additional (meth)-acryloyloxy radicals, and rings A and B are identical or different and can be aromatic or saturated.

6. Use of (meth)-acrylic acid derivatives of triisocyanates, of the formula

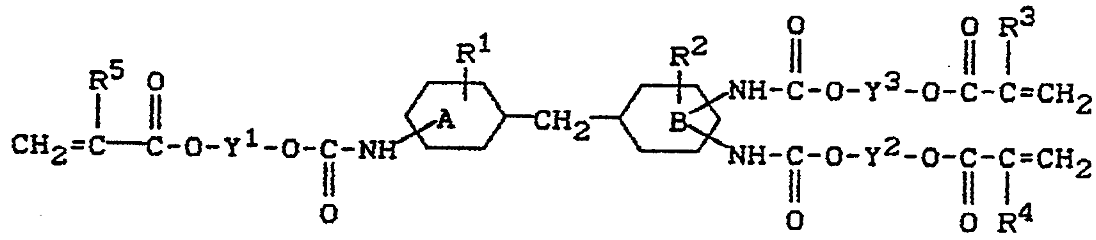

in which

$R^1$ and $R^2$ are identical or different and represent hydrogen or an alkyl radical having 1-6 carbon atoms,

$R^3$, $R^4$ and $R^5$ are identical or different and denote hydrogen or methyl,

$Y^1$ to $Y^3$ are identical or different and denote divalent straight-chain or branched aliphatic hydrocarbon radicals which have 2 to 15 carbon atoms, may optionally contain 1 to 3 oxygen bridges and can optionally be substituted by 1 to 4 additional (meth)-acryloyloxy radicals, and the rings A and B are identical or different and can be aromatic or saturated, in dental materials.

7. Use according to Claim 6, characterized in that the (meth)-acrylic acid derivatives of triisocyanates are employed in dental filling materials.

8. Use according to Claim 6, characterized in that the (meth)-acrylic acid derivatives of triisocyanates are employed in coating agents for teeth.

9. Use according to Claim 6, characterized in that the (meth)-acrylic acid derivaLtives of triisocyanates are employed for the production of plastic teeth.

10. Dental materials, characterized in that they contain (meth)-acrylic acid derivatives of triisocyanates, of the formula

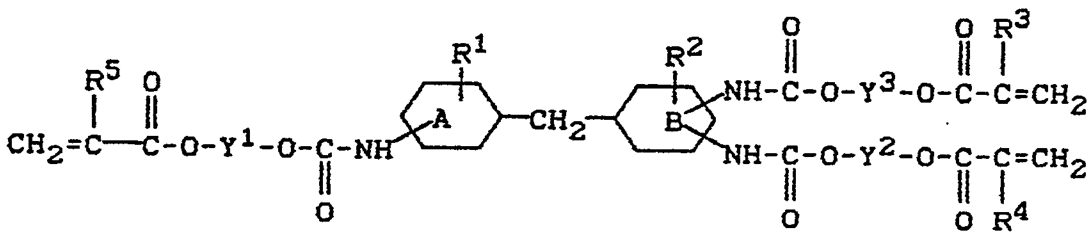

in which

$R^1$ and $R^2$ are identical or different and represent hydrogen or an alkyl radical having 1-6 carbon atoms.

$R^3$, $R^4$ and $R^5$ are identical or different and denote hydrogen or methyl,

$Y^1$ to $Y^3$ are identical or different and denote divalent straight-chain or branched aliphatic hydrocarbon radicals which have 2 to 15 carbon atoms, may option, ally contain 1 to 3 oxygen bridges and can optionally be substituted by 1 to 4 additional (meth)-acryloyloxy radicals, and the rings A and B are identical or different and can be aromatic or saturated.

**Revendications**

1. Dérivés (méth)acryliques de triisocyanates de formule

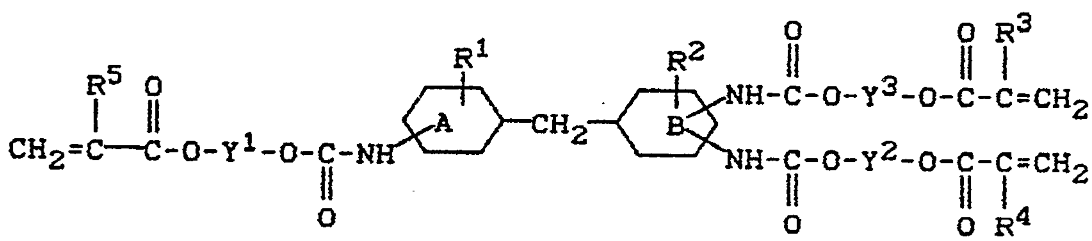

dans laquelle

$R^1$ et $R^2$, ayant des significations identiques ou différentes, représentent chacun l'hydrogène ou un groupe alkyle en $C_1$-$C_6$,

$R^3$, $R^4$ et $R^5$, ayant des significations identiques ou différentes, représentent chacun l'hydrogène ou un groupe méthyle,
$Y^1$, $Y^2$ et $Y^3$, ayant des significations identiques ou différentes, représentent chacun un radical hydrocarboné aliphatique divalent à chaîne droite ou ramifiée en $C_2$-$C_{15}$ qui peut contenir le cas échéant de 1 à 3 ponts d'oxygène et être substitué le cas échéant par 1 a 4 autres groupes (méth)acryloyloxy, et
les cycles A et B, identiques ou différents, peuvent être aromatiques ou saturés.

2. Dérivé (méth)acryliques de triisocyanates selon la revendication 1, dans lesquels
$R^1$ représente l'hydrogène,
$R^2$ représente l'hydrogène ou un groupe alkyle en $C_1$-$C_4$,
$R^3$, $R^4$ et $R^5$, ayant des significations identiques ou différentes, représentent chacun l'hydrogène ou un groupe méthyle,
$Y^1$, $Y^2$ et $Y^3$, ayant des significations identiques ou différentes, représentent chacun un radical hydrocarbone aliphatique divalent a chaîne droite ou ramifiée en $C_2$-$C_{10}$ qui peut contenir le cas échéant 1 ou 2 ponts d'oxygène et être substitué le cas échéant par 1 ou 2 autres groupes (méth)acryloyloxy,
le cycle B est aromatique ou saturé et le cyle A est saturé.

3. Dérivés (méth)acryliques de triisocyanates selon les revendications 1 et 2 dans lesquel
$R^1$ représente l'hydrogène,
$R^2$ représente l'hydrogène ou un groupe methyle,
$R^3$, $R^4$ et $R^5$, ayant des significations identiques ou différentes, représentent chacun l'hydrogène ou un groupe méthyle,
$Y^1$, $Y^2$ et $Y^3$, ayant des significations identiques ou différentes, représentent chacun un radical hydrocarboné aliphatique divalent à chaîne droite ou ramifiée en $C_2$-$C_{10}$ qui peut contenir le cas échéant 1 ou 2 ponts d'oxygène et être substitué le cas échéant par 1 ou 2 autres groupes (méth)acryloyloxy, et les cycles A et B sont saturés.

4. Un procédé de préparation des dérivés (méth)acryliques selon les revendications 1 à 3, caractérisé en ce que l'on fait réagir un triisocyanate de formule

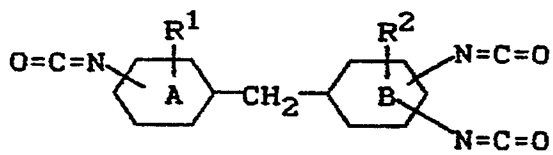

(II)

dans laquelle
$R^1$ et $R^2$, ayant des significations identiques ou différentes, representent chacun l'hydrogène ou un groupe alkyle en $C_1$-$C_6$, et les cycles A et B, identiques ou différents, peuvent être aromatiques ou saturés, avec un (méth)acrylate d'hydroxyalkyle de formules

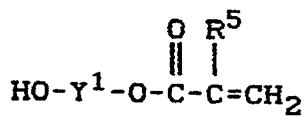

(III)

et/ou

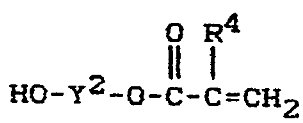

(IV)

et/ou

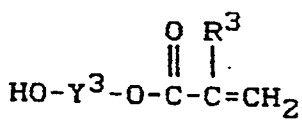

(V)

dans lesquelles
$R^3$, $R^4$ et $R^5$, ayant des significations identiques ou différentes, représentent chacun l'hydrogène ou un groupe méthyle, et

$Y^1$, $Y^2$ et $Y^3$, ayant des significations identiques ou differentes, représentent chacun un radical hydrocarbone aliphatique divalent à chaîne droite ou ramifiée en $C_2$-$C_{15}$ qui peut contenir le cas échéant 1 a 3 ponts d'oxygène et être substitué le cas échéant par 1 a 4 autres groupes (méth)acryloyloxy.

5 Polymère de dérivés (méth)acryliques de triisocyanates de formule

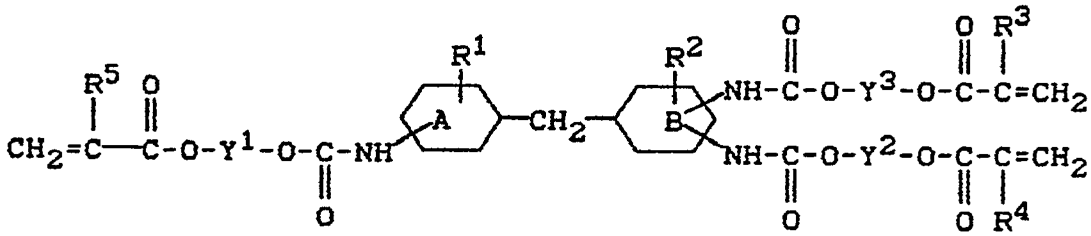

dans laquelle

$R^1$ et $R^2$, ayant des significations identiques ou différentes, représentent chacun l'hydrogène ou un groupe méthyle,

$Y^1$, $Y^2$ et $Y^3$, ayant des significations identiques ou différentes, représentent chacun un radical hydrocarboné aliphatique divalent à chaîne droite ou ramifiée en $C_2$-$C_{15}$ qui peut contenir le cas échéant 1 a 3 ponts d'oxygène et être substitué le cas échéant par 1 à 4 autres groupes (méth)acryloyloxy, et les cycles A et B, identiques ou différents, peuvent être aromatiques ou saturés.

6. Utilisation des dérivés (méth)acryliques de triisocyanates de formule

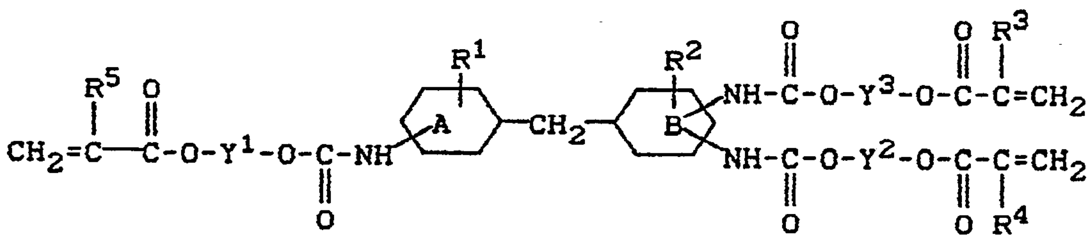

dans laquelle

$R^1$ et $R^2$, ayant des significations identiques ou différentes, représentent chacun l'hydrogène ou un groupe alkyle en $C_1$-$C_6$,

$R^3$, $R^4$ et $R^5$, ayant des significations identiques ou différentes, représentent chacun l'hydrogène ou un groupe méthyle,

$Y^1$, $Y^2$ et $Y^3$, ayant des significations identiques ou différentes, représentent chacun un radical hydrocarbone aliphatique divalent à chaîne droite ou ramifiée en $C_2$-$C_{15}$ qui peut contenir le cas échéant 1 à 3 ponts d'oxygène et être substitué le cas échéant par 1 a 4 autres groupes (méth)acryloyloxy, et les cycles A et B, identiques ou différents, peuvent être aromatiques ou saturés, dans des matériaux dentaires.

7. Utilisation selon la revendication 6, caractérisée en ce que les dérivés (meth)acryliques de triisocyanates sont utilisés dans des masses d'obturation dentaires.

8. Utilisation selon la revendication 6, caractérisée en ce que les dérivés (méth)acryliques de triisocyanates sont utilisés dans des produits de revêtement pour les dents.

9. Utilisation selon la revendication 6, caractérisée en ce que les dérivés (méth)acryliques de triisocyanates sont utilisés pour la formation de dents artificielles.

10. Matériaux dentaires, caractérisés en ce qu'ils contiennent des dérivés (méth)acryliques de triisocyanates de formule

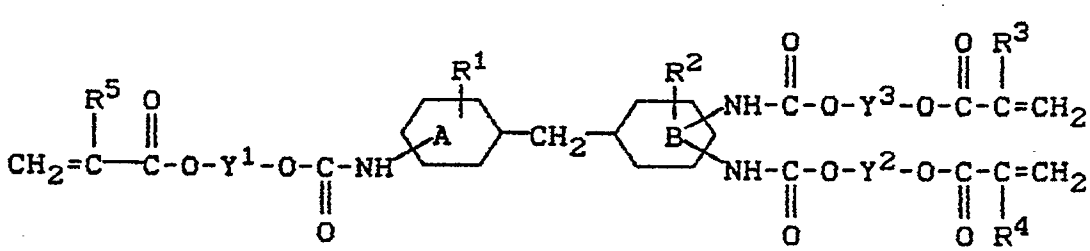

dans laquelle

$R^1$ et $R^2$, ayant des significations identiques ou différentes, représentent chacun l'hydrogène ou un groupe alkyle en $C_1$-$C_6$,

$R^3$, $R^4$ et $R^5$, ayant des significations identiques ou différentes, représentent chacun l'hydrogène ou un groupe méthyle,
$Y^1$, $Y^2$ et $Y^3$, ayant des significations identiques ou différentes, représentent chacun un radical hydrocarboné aliphatique divalent à chaîne droite ou ramifiée en $C_2$-$C_{15}$ qui peut contenir le cas échéant 1 a 3 ponts d'oxygène et être substitué le cas échéant par 1 à 4 autres groupes (méth)acryloyloxy, et les cycles A et B sont identiques ou différents et peuvent être aromatiques ou saturés.